# EUROPEAN PATENT SPECIFICATION

(11) **EP 1 200 139 B1**
(45) Date of publication and mention of the grant of the patent: **29.10.2003**
(21) Application number: 00952066.9
(22) Date of filing: 07.08.2000
(51) Int. Cl.: A61L 31/06

(54) **FIXATIVE DEVICE**
FESTIGUNGSVORRICHTUNG
DISPOSITIF DE FIXATION

(30) Priority: 06.08.1999 EP 99202597
(43) Date of publication of application: 02.05.2002
(73) Proprietor: IsoTis N.V., 3723 MB Bilthoven (NL)
(72) Inventor: BEZEMER, Jeroen, Mattijs, NL-3562 VG Utrecht (NL); DE WIJN, Joost, Robert, NL-6522 BP Nijmegen (NL); NIEUWENHUIS, Jan, NL-4207 KD Gorinchem (NL); DE JOODE, Paul, Carel, Hubertus, NL-3911 AS Rhenen (NL); VAN BLITTERSWIJK, Clemens, Antoni, NL-3467 PD Hekendorp (NL)
(74) Representative: Prins, Adrianus Willem
(86) International application number: NL0000555
(87) International publication number: WO01010480

(56) References cited:
- EP-A- 0 357 155
- EP-A- 0 891 783
- WO-A-95/29201
- US-A- 5 312 435

## Description

The invention relates to a fixative device for attachment of tissues, in particular for attachment of soft tissue to bony tissue.

In surgery, many different fixative devices for tissue are used. Well-known examples include sutures, bone screws, suture anchors, clips, pins, staples, and the like.

Bone screws are widely used as temporary medical implants for the fixation of skeletal fractures and/or for the fixation of orthopaedic implants. Important areas of application are fixation of the spinal cord after a geometric correction, healing of fractures in the knee, heel, elbow or hip, and fixation of hip prostheses. Bone screws are generally made of a metallic material, e.g. of titanium, cobalt-chromium alloys, and stainless steel. Recently, however, some biodegradable, synthetic polymers, such as polymers of lactic acid, have been employed for manufacturing bone screws. An important property the materials should possess, is an excellent biocompatibility in contact with bone and surrounding tissues.

Suture anchors are devices which are increasingly used, particularly in trauma and sports surgery, for fixing soft tissue, such as muscle or ligament tissue, to hard tissues, such as (cortical) bone tissue. These devices generally consist of two parts. One part is a screw-like device, e.g. a bone screw, which is fixed to the hard tissue by providing a cavity in the hard tissue and by tapping screw thread in the sidewalls of the cavity for cooperation with the screw thread of the screw bone screw; the other is a device which is attached to the screw-like device and may be connected to soft tissue. Often, the latter device is a suture-like material made of a multifilament or monofilament, biodegradable material, such as Biosyn™, Monocryl™, Vicryl™ or Dexon™.

Another manner of attaching soft tissue to bony tissue concerns the use of the Anterior Cruciate Ligament (ACL) system. To this end, a hole is drilled in the tibia and/or femur, in the sidewalls of which screw thread is subsequently tapped. In this hole, the ligament is placed, and fixed in position by a screw which is applied over the ligament.

Recently, there has been a trend to use a biodegradable material, instead of a metallic material, for the manufacture of the above fixative devices. Examples of proposed biodegradable materials in this regard are polyglycolides, polylactides and blends or copolymers thereof. The great advantage of the devices being of a biodegradable material is that the material decomposes after a predetermined period of time. This means that it is not necessary to remove the device when its presence is no longer needed, and that a surgical removal procedure may be omitted. Of course, the rate of degradation of the material should be chosen such that sufficient mechanical strength is provided by the device until the presence of the device is no longer needed.

As has been indicated above, the connection of the various fixative devices to bone tissue is generally based on the use of screw-thread. A serious disadvantage of this manner of connecting the device to bone tissue, is that the cavity, into which the device is to be fixed, needs to be provided with screw-thread in order to provide the connection with sufficient mechanical strength. The tapping of screw thread constitutes an additional surgical step to be performed, leaving bony debris at the site of implantation. Generally, small defects at the site of implantation, which are the result of the rather rigorous procedure of tapping screw thread, may be invaded by fibrous tissue, which might be guided through the screw canal.

The present invention aims to provide a new type of fixative device, which may be fixed to bone tissue without the need for prethreading the cavity by tapping screw-thread in the bone tissue. It shall be clear that the word cavity in this context is meant to comprise orifices and other types of holes.

It is further an object of the invention to provide a fixative device having sufficient mechanical strength, and which device may provide a connection to bone tissue having sufficient mechanical strength, so that the device may be used in various surgical procedures. Another object of the invention is to provide a fixative device for bone tissue, which is made of a biocompatible and biodegradable material.

It has been found that the above goals are reached by using a specific class of copolymers for the manufacture of a fixative device. Surprisingly, the class of copolymers shows a favorable swelling behavior, which behavior may be used to fix a device, based on a member of said class of copolymers, to bone tissue without the use of screw-thread. This specific class of copolymers is formed by copolymers of a polyalkylene glycol terephtalate and an aromatic ester.

Accordingly, the invention relates to a fixative device for bone tissue, which device comprises a copolymer of a polyalkylene glycol terephtalate and an aromatic polyester.

The specific class of copolymers, on which the present device is based, shows a highly favorable swelling behavior in an aqueous environment. The swelling may be as high as approximately 5-100% by volume. The swelling of the copolymer leads to a swelling of the device, which results in a very strong fixation of the device to e.g. bone tissue.

To connect the fixative device to the bone tissue, the device is simply inserted into a cavity provided in the bone, and the swelling causes such expansion of the device that it becomes jammed in the cavity. It has been found that the mechanical strength of the fixation thus provided is sufficient for various purposes in surgical treatment. Accordingly, the tapping of screw-thread in a cavity in bone tissue in which the device is to be fixed is not necessary.

Further, the copolymer is biodegradable. In fact, the biodegradability (the rate of degradation under certain conditions) may be controlled, depending on the envisaged application of the device.

The copolymer on which the present device is based, is a copolymer of a polyalkylene glycol terephtalate and an aromatic polyester. Preferably, the copolymer comprises 20-90 wt.%, more preferably 40-70 wt.% of the polyalkylene glycol terephtalate, and 80-10 wt.%, more preferably 60-30 wt.% of the aromatic polyester. A preferred type of copolymers according to the invention is formed by the group of block copolymers.

The polyalkylene glycol terephtalate may have a weight average molecular weight of about 150 to about 4000. Preferably, the polyalkylene glycol terephtalate has a weight average molecular weight of 200 to 1500. The aromatic polyester preferably has a weight average molecular weight of from 200 to 5000, more preferably from 250 to 4000. The weight average molecular weight of the copolymer preferably lies between 10,000 and 300,000, more preferably between 40,000 and 120,000.

The weight average molecular weight may suitably be determined by gel permeation chromatography (GPC). This technique, which is known per se, may for instance be performed using chloroform as a solvent and polystyrene as external standard. Alternatively, a measure for the weight average molecular weight may be obtained by using viscometry (see NEN-EN-ISO 1628-1). This technique may for instance be performed at 25°C using chloroform as a solvent. Preferably, the intrinsic viscosity of the copolymer lies between 0.2289 and 1.3282 dL/g, which corresponds to a weight average molecular weight between 10,000 and 200,000. Likewise, the more preferred ranges for the weight average molecular weight measured by GPC mentioned above can also be expressed in terms of the intrinsic viscosity.

In a preferred embodiment, the polyalkylene glycol terephtalate component has units of the formula -OLO-CO-Q-CO-, wherein O represents oxygen, C represents carbon, L is a divalent organic radical remaining after removal of terminal hydroxyl groups from a poly(oxyalkylene)glycol, and Q is a divalent organic radical.

Preferred polyalkylene glycol terephtalates are chosen from the group of polyethylene glycol terephtalate, polypropylene glycol terephtalate, and polybutylene glycol terephtalate and copolymers thereof, such as poloxamers. A highly preferred polyalkylene glycol terephtalate is polyethylene glycol terephtalate.

The terms alkylene and polyalkylene generally refer to any isomeric structure, i.e. propylene comprises both 1,2-propylene and 1,3-propylene, butylene comprises 1,2-butylene, 1,3-butylene, 2,3-butylene, 1,2-isobutylene, 1,3-isobutylene and 1,4-isobutylene (tetramethylene) and similarly for higher alkylene homologues. The polyalkylene glycol terephtalate component is preferably terminated with a dicarboxylic acid residue -CO-Q-CO-, if necessary to provide a coupling to the polyester component. Group Q may be an aromatic group having the same definition as R, or may be an aliphatic group such as ethylene, propylene, butylene and the like.

The polyester component preferably has units -O-E-O-CO-R-CO-, wherein O represents oxygen, C represents carbon, E is a substituted or unsubstituted alkylene or oxydialkylene radical having from 2 to 8 carbon atoms, and R is a substituted or unsubstituted divalent aromatic radical.

In a preferred embodiment, the polyester is chosen from the group of polyethylene terephthalate, polypropylene terephthalate, and polybutylene terephthalate. A highly preferred polyester is polybutylene terephthalate.

The preparation of the copolymer will now be explained by way of example for a polyethylene glycol terephtalate/polybutylene terephthalate copolymer. Based on this description, the skilled person will be able to prepare any desired copolymer within the above described class. An alternative manner for preparing polyalkylene glycol terephtalate/polyester copolymers is disclosed in US-A-3,908,201.

A polyethylene glycol terephtalate/polybutylene terephthalate copolymer may be synthesized from a mixture of dimethyl terephthalate, butanediol (in excess), polyethylene glycol, an antioxidant and a catalyst. The mixture is placed in a reaction vessel and heated to about 180°C, and methanol is distilled as transesterification proceeds. During the transesterification, the ester bond with methyl is replaced with an ester bond with butylene and/or the polyethyene glycol. After transesterification, the temperature is raised slowly to about 245°C, and a vacuum (finally less than 0.1 mbar) is achieved. The excess butanediol is distilled off and a prepolymer of butanediol terephthalate condenses with the polyethylene glycol to form a polyethylene/polybutylene terephthalate copolymer. A terephthalate moiety connects the polyethylene glycol units to the polybutylene terephthalate units of the copolymer and thus such a copolymer also is sometimes referred to as a polyethylene glycol terephthalate/polybutylene terephthalate copolymer (PEGT/PBT copolymer).

Depending on the specific circumstances under which a fixative device according to the invention is intended to be used, the copolymeric material described above may be used in either a porous or a dense, nonporous form. A porous structure enables ingrowth of tissue, for instance of bone tissue. In general, when the fixative device is a suture anchor this type of tissue ingrowth is desired. The skilled person will be able to judge under which circumstances a porous or a dense structure will be preferred.

Optionally, the above copolymer may be combined with other materials in the manufacture of a fixative device according to the invention. It may for instance be advantageous to provide a material that facilitates bone ingrowth at the distal end of the device that fits into the bone. Examples of such a material include metals, such as tantalum or Ti6Al4V, or ceramics, such as calcium phosphates. Preferably, the material is a porous metal. In a preferred embodiment, this material may comprise a coating, e.g. as described in the European patent application 98203085.0. Thus, in accordance with this embodiment, the device is composed of different parts which are attached to each other.

The device in accordance with this embodiment may be assembled by injection molding the copolymeric material onto the material that facilitates bone ingrowth. Particularly when the latter is a porous material, the melt of the copolymer may penetrate the porous structure of said material. Generally, it will be preferred that the copolymer penetrates into the porous structure to a depth of 1-5 mm, more preferably 3-4 mm, depending on the size of the device to be manufactured and its specific application.

A fixative device for bone tissue according to the present invention may be used in any application wherein conventional fixative devices for bone tissue are applied. As has been mentioned above, one of the great advantages of the invention is that it is not necessary to provide a cavity in bone tissue with screw-thread in order to fix the present device to the bone tissue. The swelling behavior of the device provides sufficient mechanical strength for the device to be used without the use of screw-thread. Furthermore, the specific copolymer on which the present fixative device is based, is capable of actively providing a bond with bone tissue.

In order to fix a device according to the invention to bone tissue, the device should be in a non-swollen condition. Preferably, the device is in a substantially moist-free condition when applied to bone. It is preferred that the device has a moisture content of less than 1%, more preferably less than 0.1%. These low moisture contents further have been found to have a favorable effect on the shelf life of the device.

Once the device has been placed into a cavity in bone tissue, the device is wetted in order to make it swell. Due to the presence of body and wound fluids surrounding the site of implantation of the device, the device takes up moisture and may swell to an extent of up to 5-100% of its original volume. The increase in volume fixes the device in its place. In particular, expansion of the device in the cavity causes increased pressure on the walls thereof. This in turn increases friction between the walls of cavity and the device, which provides increased mechanical resistance, and thus increased mechanical strength.

Advantageously, it has been found that the fixation of the device thus obtained is able to withstand a force up to or higher than the breaking strength of common suture threads, e.g. a force up to or higher than 200 N.

Using a copolymer described hereinabove, any fixative device for bone tissue may be manufactured. Examples of fixative devices include bone screws, suture anchors, staples, both tapered and non-tapered pins, and so forth. In principle, any type of fixative device may be manufactured which device may be fixed to human tissue due to its swelling behavior.

The invention will now be elucidated on the basis of two preferred embodiments, shown in a drawing. In the drawing:
fig. 1 shows a first embodiment of a fixative device according to the invention fixating an anterior cruciate ligament to a tibia and a femur;
figs. 2A and 2B show a plan and side view of a second embodiment of a fixative device according to the invention respectively;
fig. 3 shows the fixative device according to figs. 2 connecting a ligament to a bone;
Fig. 4 shows a partial cross section of a third embodiment of a fixative device according to the invention fixating an anterior cruciate ligament to a tibia and a femur;
Fig. 4A shows a plan view of the ligament ends tied in a knot around the fixative device of Fig. 4;
Fig. 4B shows a cross section of the fixative device of Fig. 4;
Fig. 4C shows a plan view of the ligament ends before being tied in a knot around the fixative device of Fig. 4;
Fig. 5A shows a perspective view of a reinforcing rod and Figs. 5B to 5D a cross sectional view of a bone having a cavity in which the reinforcing rod and the fixative device is inserted;
Fig. 6A shows a perspective view of a fourth embodiment of a fixative device; and
Fig. 6B shows a cross sectional view of a bone in which the fixative device of Fig. 6A has been inserted.

The figures are schematic representations of preferred embodiments of the invention and serve as illustrations only. In the figures, identical or corresponding parts are designated by the same reference numerals.

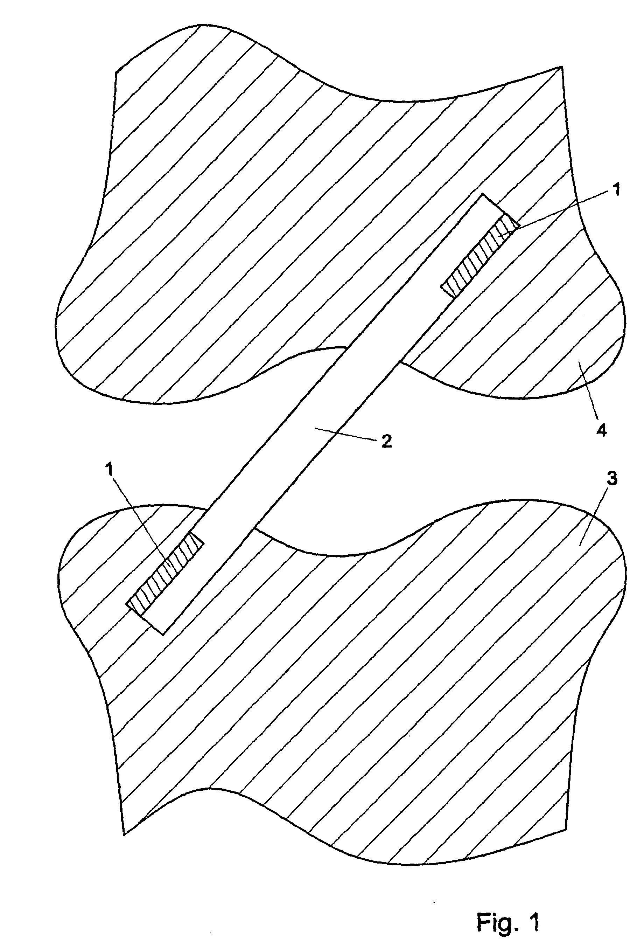

Figure 1 shows a first embodiment of the invention wherein the fixative devices 1 are used for fixation of an anterior cruciate ligament 2 to the tibia 3 and femur 4. Holes have been drilled in both the tibia 3 and the femur 4. In at least one of these holes, the fixative devices 1 which are constructed as expansion bodies are fitted in dry, unswollen state together with the anterior cruciate ligament 2. The fixative devices 1 will swell up due to the presence of body fluids. If desired, the swelling may be facilitated or accelerated by provision of additional moisture. The swelling will jam the anterior cruciate ligament 2 in the holes provided in the tibia 3 and the femur 4, thus holding the anterior cruciate ligament 2 in place.

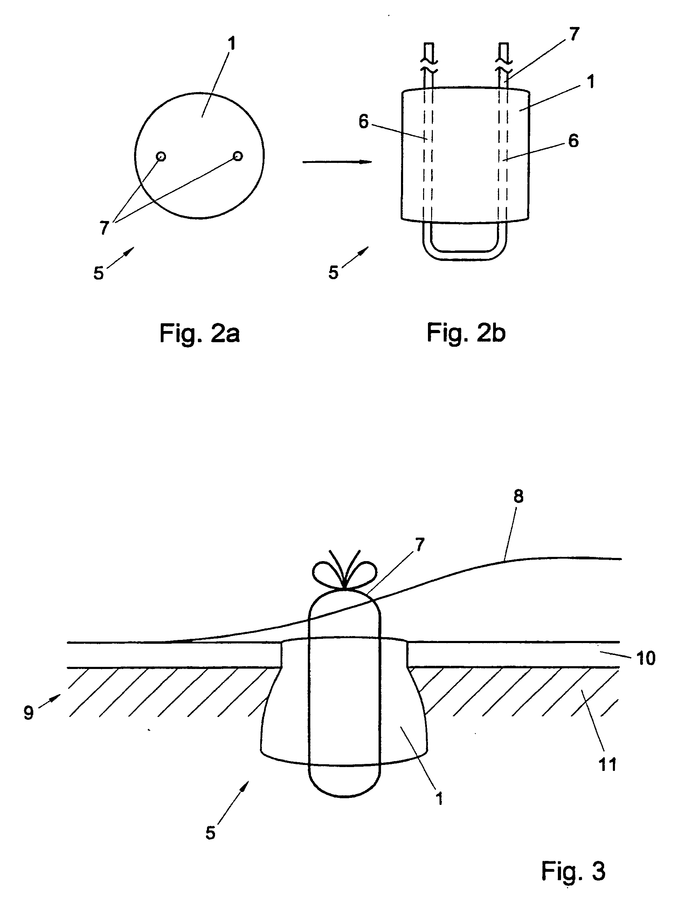

Figures 2 and 3 show a second embodiment of the fixative device 1 as a suture anchor 5. The suture anchor comprises an expansion body 1 of substantially cylindrical shape. The expansion body 1 comprises two bores or channels 6 through which a suture thread 7 is provided. This suture thread 7 is preferably made of biodegradable material, for instance also of a copolymer of a polyalkylene glycol terephtalate and an aromatic ester. As has been mentioned above, the expansion body 1 could be composed of two different materials, the lower part being made of the copolymer of a polyalkylene glycol terephtalate and an aromatic ester, and the upper part of a material that facilitates bone ingrowth, such as (porous) tantalum.

In order to attach a ligament 8 to a bone 9, first a hole is drilled in the bone 9 through the cortical bone 10 into the spongy bone 11. In this hole, the suture anchor 5 is placed. The suture thread 7 of the suture anchor is used to fix the ligament 8 to the bone, for instance by means of a knot. Due to the presence of moisture, the cylindrical expansion body 1 of the suture anchor will swell and fix the device into place. As the copolymer expands easily in the compressible spongy bone surroundings than in the firm cortical bone surroundings, the suture anchor will become wedged firmly in place.

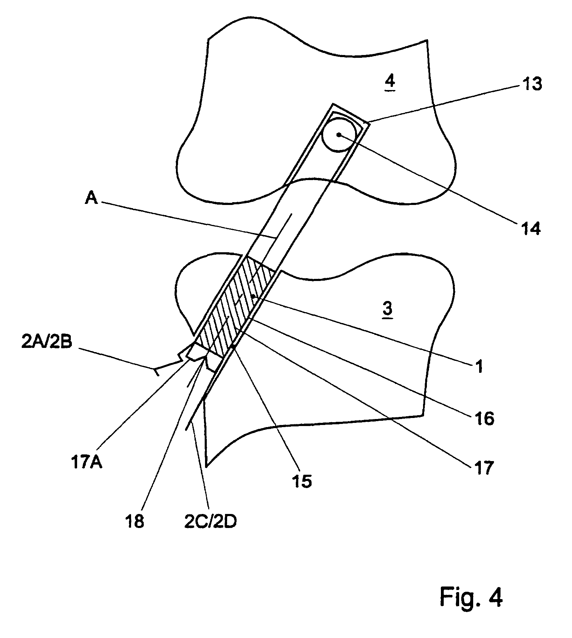

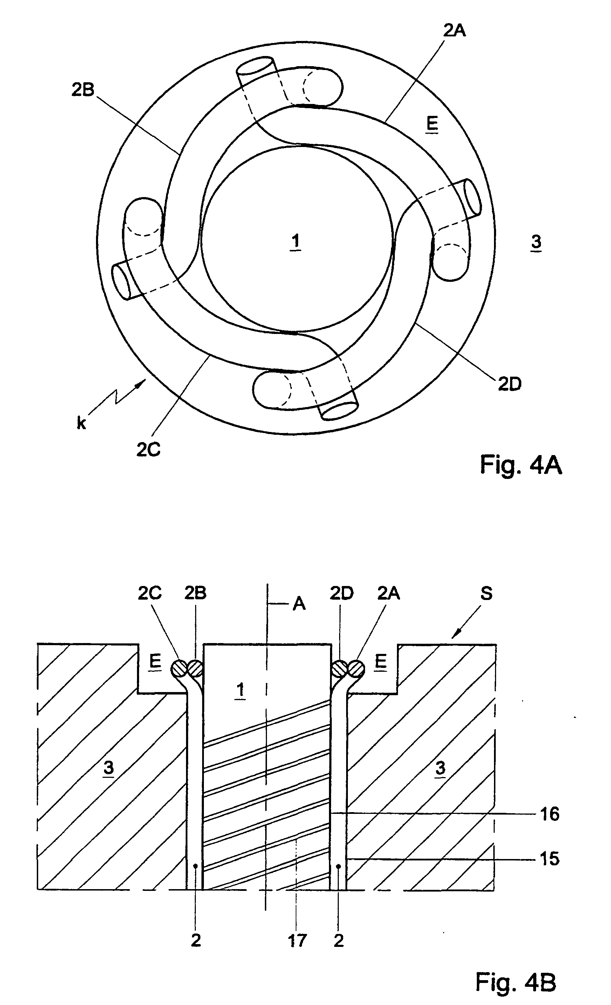

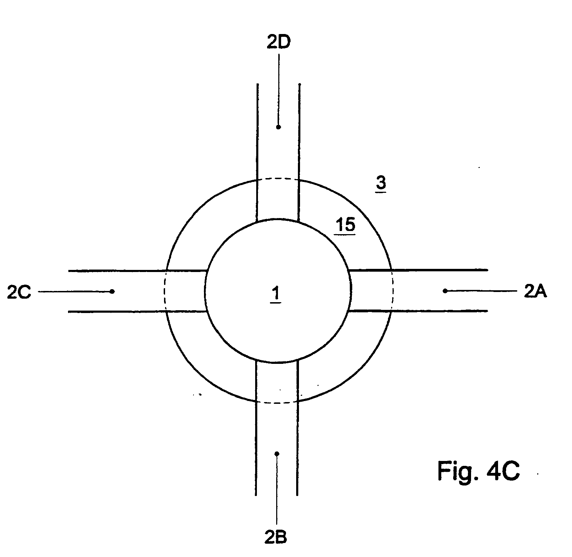

Fig. 4 shows a third embodiment wherein the fixative device 1 is used as an ACL plug for fixation of an anterior cuciate ligament (ACL) 2. The ligament 2 is connected to the femur 4 in a conventional manner, e.g. the cruciate ligament 2 extends through a hole 13 drilled in the femur 4 and comprises two tendons which are looped over a pin 14 extending transversely through the hole 13, such that the ligament 2 is anchored to the femur 4.

The ligament 2 which comprises the four end portions 2A-2D of the looped tendons extends through a hole 15 which is drilled in the tibia 3. To secure the ligament ends 2A-D in the tibial hole 15, a fixative device 1 is introduced. The fixative device 1 comprises an elongated, substantially cylindrical body extending along a central axis A, having a portion of is outer surface 16 forming the cylinder mantle provided with screw thread 17. The fixative device 1 is made of a copolymer of a polymer alkylene and aromatic polyester. The screw thread 17 has a pitch extending in the direction of axis A, such that, even if the fixative device is made of relatively flexible material, insertion into the tibial hole 15 can be performed relatively easily. The fixative device 1 is provided with a head portion 17A with a drive surface for engagement with a tool for rotationally driving the fixative device about the axis A of the tibial hole 15. The drive surface may e.g. comprise a screw type serration 18 for engagement by a screw driver or a square or hexagonal head for engagement by e.g. a wrench. By providing the screw thread 17 with blunt edges or with a square cross section, the chance of damaging the ligament ends 2A-2D can be reduced.

The free ends 2A-2D of the ligament 2 may be secured to the fixative device by a knot K. In particular, the knot K can be formed by looping each free end around the mantle of fixative device 1 such that it is interposed between the mantle of the fixative device and an adjacent ligament end in clockwise or counter clockwise direction as is shown in Fig. 4A. After securing them to the fixative device, the loose ends of the ligaments 2A-2D may be cut off as shown. Preferably, the tibial hole 15 is provided with an enlarged entrance portion E for accommodating the knot K and/or a head portion 17A having an enlarged diameter relative to the elongate body. This way it can be achieved that the knot K does not extend outwardly relative to the bone 3 and the skin surface can remain smooth. In an advantageous manner, any portion of the fixative device 1 protruding beyond the bone surface S, e.g. the head portion 17, may be cut off to the surface level of the bone as indicated in Fig. 4B. By tying the free ligament ends 2A-2D in a knot K around the mantle surface 16 of the fixative device 1 the need for additional clamps is obviated.

A presently most preferred way of tying the free ends 2A-2D in a knot K around the fixative device 1 is in a reef knot as discussed in relation to Fig. 4C. First, free end 2A is moved clockwise to extend over free end 2B (to the bottom left of Fig. 4C). Next, free end 2B is moved clockwise to extend over free end 2A and 2C (to the top left of Fig. 4C). Subsequently, free end 2C is moved clockwise over free ends 2B and 2D (to the top right of Fig. 4c). Finally, free end 2D is moved clockwise to extend over free end 2A (to the bottom right of Fig. 4C) and is tucked in between the mantle 16 of the fixative device and the free end 2A and is pulled tight.

It shall be clear that the above described method for tying the free ends of the ligaments to the fixative device extending from the tibial hole may also be used in combination with fixative devices that are made of non-swellable, biocompatable material e.g. titanium.

As the fixative device 1 is introduced in dry, unswollen state it will initially fix the ligament ends 2A-2D to the walls of the tibial hole 15 by means of increased friction due to contact pressure. After introduction, the fixative device 1 will pick up fluid from its natural surroundings and/or from additional moistening.

The resulting swelling will further press the ligament ends 2A-2D against the bony wall of the tibia hole 15. At a later stage, the bone material and the material of the fixative device may grow into contact with each other and the material of the fixative device may be replaced by bone material due to bone ingrowth.

To enhance ingrowth of bone material, the fixative device 1 may be formed as a composite including calcium phosphate. In particular, the calcium phosphate may be applied as a coating as discussed in European patent application 99202281.4 and/or as a mixture as disclosed in European patent application 99203141.9, the text of which applications is herein incorporated by reference.

It shall be clear that this embodiment may also be used to fix other types of ligaments or sutures to the walls of an aperture.

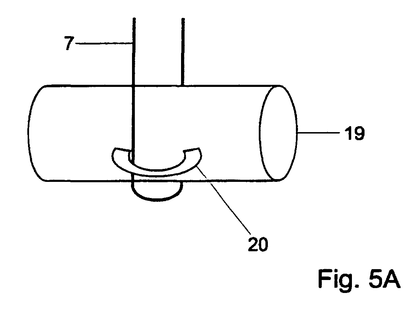

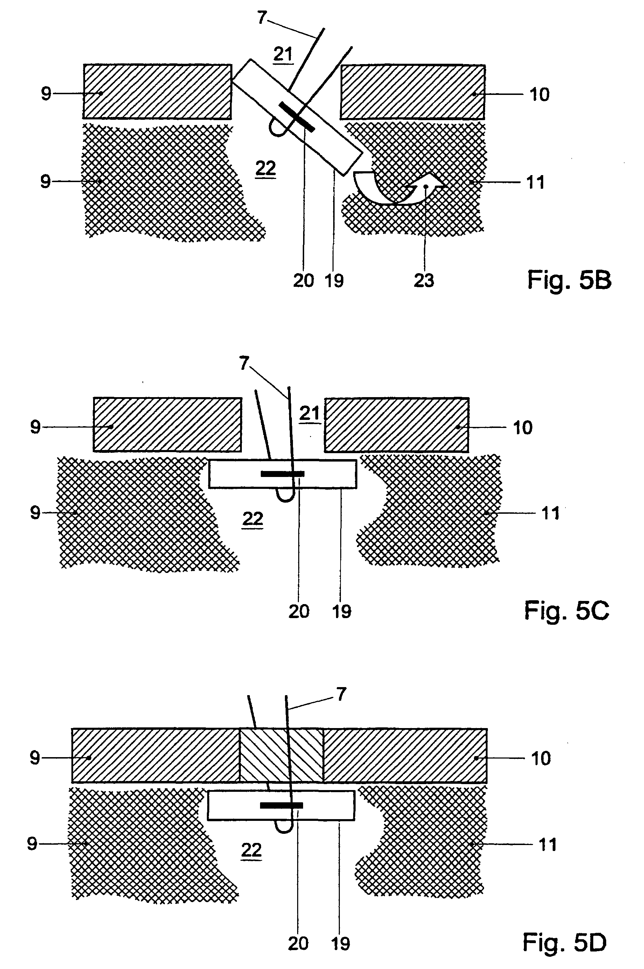

Figures 5A-5D show that the fixative device 1 may be used in combination with a supporting rod 19 made of stiff and tear resistant material. The supporting rod 19 may be provided with loops 20 to guide the suture 7 along the supporting rod 19. Fig. 5B shows that the supporting rod 19 with the attached suture thread 7 may be introduced in a tilted position through a hole 21 in the bone 9 into an underlying larger cavity 22 in the bone 9. The hole 21 and the underlying undercut cavity 22 can be made by a surgeon using a suitable instrument, e.g. a cylindrical or spherical burr. After insertion, the supporting rod 19 is tilted in the direction of arrow 23, such that it extends transversely in the hole 22 as shown in Fig. 5C. When a pulling force is applied to the ends of the suture thread 7, the supporting rod 19 is locked into place as its length is chosen larger than the diameter of the hole 21. The hole 21 is subsequently closed by a fixative device 1, such that the hole is locked.

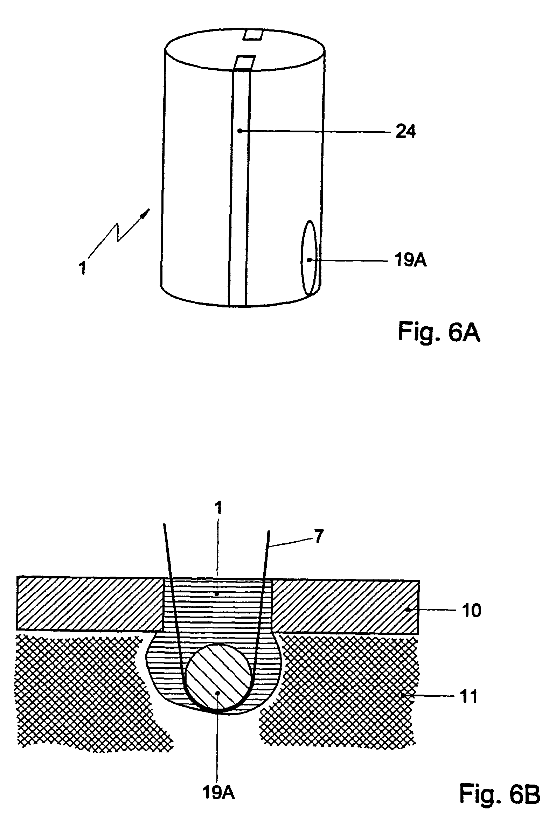

Figs. 6A and 6B show yet another embodiment of the fixative device 1. The fixative device 1 comprises a substantially cylindrical body of swellable material, which has been provided with longitudinal grooves 24 in its mantle surface to guide the suture thread 7. The fixative device 1 is further provided with a reinforcing portion 19A for distributing the pressure applied by the suture thread 7. The reinforcing portion 19A comprises a material having a relatively high stiffness and tear resistance, which may exhibit a lower degree of swelling. The reinforcing portion is preferably cylindrical, such that the pressure exerted by the suture thread 7 can be distributed evenly. As shown in cross section in Fig. 6B, the suture thread 7 can still be moved through the slots 24 and over the reinforcing portion in a sliding manner. The fixative device 1 itself is locked in the hole in a manner as described in relation to Fig. 3.

It will be clear that the invention is not limited to the described preferred embodiments and merely show the principles underlying the invention. For example, the expansion body may have various shapes and/or surface textures, e.g. cylindrical, frusto conical or block-shaped. In addition, the shape of the expansion body may vary at different locations, e.g. a cylindrical body may be provided with a convex tip. Furthermore, the cavity of the bone into which the fixative device is to be inserted, may be provided with undercuts to increase the immediate anchoring capacity of the fixative device.

In addition, the fixative device may be provided with protrusions or areas of increased swelling capacity to increase the anchoring or jamming function. Furthermore, the expansion body may be connected to various other types of sutures, e.g. staples or clamps. In addition, the fixative device may comprise a number of swelling bodies and the fixative device may be provided with connecting means, e.g. a socket or similar means for accommodating objects or e.g. an arm carrying a connector.

Such embodiments are within the scope of the invention as defined in the appended claims.

The invention will now be further elucidated by the following, non-restrictive example.

### EXAMPLE

A cylinder (diameter 1.5 cm, height 3 cm) of a copolymer of polyethylene glycol terephtalate (Mw = 574) and polybutylene terephtalate (Mw = 800), comprising 60 wt.%, based on the weight of the copolymer, of the polyethylene glycol terephtalate, was provided with two holes in axial direction (diameter 0.5 mm). Through these holes, a Vicryl™ mulitfilament thread (length 0.5 m) was provided to obtain a device as shown in Figure 3.

In a bovine femur, a hole was drilled through the cortical bone into the spongy bone of a diameter of 1.55 cm. The device was fitted into this hole in a dry state (moisture content below 0.5%). Next, the device was wetted using 100 ml water. As a result, the device swelled and was fixed to the bone. After 12 hours, a force of around 100 N was applied to the thread without the device showing any movement in the hole.

## Claims

1. A fixative device for bone tissue, which device comprises a copolymer of a polyalkylene glycol terephtalate and an aromatic polyester.

2. A device according to claim 1, wherein the aromatic polyester is formed from an alkylene glycol terephtalate having from 2 to 8 carbon atoms and an aromatic dicarboxylic acid.

3. A device according to claim 1 or 2, wherein the polyalkylene glycol terephtalate is chosen from the group of polyethylene glycol terephtalate, polypropylene glycol terephtalate, polybutylene glycol terephtalate, and copolymers thereof.

4. A device according to any of the preceding claims, wherein the polyester is chosen from the group of poly(ethyleneterephtalate), poly(propyleneterephtalate), and poly(butyleneterephtalate).

5. A device according to any of the preceding claims, wherein the copolymer is a polyethylene glycol terephtalate/poly(butyleneterephtalate) copolymer.

6. A device according to any of the preceding claims, wherein the copolymer comprises 20-90 wt.%, preferably 40-70 wt.%, of the polyalkylene glycol terephtalate.

7. A device according to any of the preceding claims, wherein the polyalkylene glycol terephtalate has a weight average molecular weight of from 150 to 4000, preferably from 200 to 1500.

8. A device according to any of the preceding claims, further comprising a material that facilitates bone ingrowth.

9. A fixative device according to any of the preceding claims, comprising at least one swelling body including said copolymer.

10. A fixative device according to claim 8, further comprising connecting means for connecting objects thereto.

11. A device according to any of the preceding claims, being a bone screw, a suture anchor, an ACL plug, a tapered or non-tapered pin, or a staple.

12. A device according to any of the preceding claims, comprising an elongate body portion extending along a central axis, the elongate body portion being on an outer surface thereof provided with screw thread, the pitch of the screw thread extending in axial direction.

13. A device according to claim 12, wherein the elongate body carries a head portion that is provided with at least one drive surface for engaging a driving tool.

14. A suture anchor according to any of claims 9-13, comprising a biodegradable multifilament or monofilament for connection to muscle or ligament tissue.

## Patentansprüche

1. Fixiervorrichtung für Knochengewebe, welche Vorrichtung ein Copolymer von Polyalkylenglycolterephthalat und einem aromatischen Polyester umfasst.

2. Vorrichtung gemäß Anspruch 1, worin der aromatische Polyester aus einem Alkylenglycolterephthalat mit zwei bis acht Kohlenstoffatomen und einer aromatischen Dicarbonsäure gebildet ist.

3. Vorrichtung gemäß Anspruch 1 oder 2, worin das Polyalkylenglycolterephthalat ausgewählt ist aus der Gruppe Polyethylenglycolterephthalat, Polypropylenglycolterephthalat, Polybutylenglycolterephthalat und Copolymeren derselben.

4. Vorrichtung gemäß einem der vorstehenden Ansprüche, worin der Polyester ausgewählt ist aus der Gruppe Poly(ethylenterephthalat), Poly(propylenterephthalat) und Poly(butylenterephthalat).

5. Vorrichtung gemäß einem der vorstehenden Ansprüche, worin das Copolymer ein Copolymer von Polyethylenglycolterephthalat und Poly(butylenterephthalat) ist.

6. Vorrichtung gemäß einem der vorstehenden Ansprüche, worin das Copolymer 20-90 Gew.-%, vorzugsweise 40-70 Gew.-% des Polyalkylenglycolterephthalats umfasst.

7. Vorrichtung nach einem der vorstehenden Ansprüche, worin das Polyalkylenglycolterephthalat ein gewichtsmittleres Molekulargewicht von 150 bis 4000, vorzugsweise von 200 bis 1500 aufweist.

8. Vorrichtung gemäß einem der vorstehenden Ansprüche, zusätzlich umfassend ein Material, welches das Einwachsen von Knochen erleichtert.

9. Fixiervorrichtung nach einem der vorstehenden Ansprüche, umfassend mindestens einen quellenden Körper, umfassend das Copolymer.

10. Fixiervorrichtung gemäß Anspruch 8, zusätzlich umfassend Anschluss- bzw. Verbindungsmittel zum Anschließen von Objekten an dieselbe.

11. Vorrichtung gemäß einem der vorstehenden Ansprüche, bei der es sich um eine Knochenschraube, einen Anker für Nahtmaterial, einen ACL-Pfropfen, eine sich verjüngende oder sich nicht verjüngende Nadel oder eine Klammer handelt.

12. Vorrichtung nach einem der vorstehenden Ansprüche, umfassend einen länglichen Körperteil, der sich entlang einer Zentralachse erstreckt, wobei eine äußere Oberfläche des länglichen Körperteils mit einem Schraubengewinde versehen ist, wobei die Teilung des Schraubengewindes sich in axialer Richtung erstreckt.

13. Vorrichtung gemäß Anspruch 12, worin der längliche Körper einen Kopfteil trägt, der mit mindestens einer Antriebsoberfläche versehen ist zum Einrasten lassen eines Antriebswerkzeuges.

14. Anker für Nahtmaterial gemäß einem der Ansprüche 9-13, umfassend ein biologisch abbaubares Multifilament oder Monofilament zur Verbindung an Muskel- oder Sehnengewebe.

## Revendications

1. Un dispositif de fixation pour du tissu osseux, lequel dispositif comprend un copolymère d'un téréphtalate polyalkylène glycol et d'un polyester aromatique.

2. Un dispositif selon la revendication 1, dans lequel le polyester aromatique est formé d'un téréphtalate alkylène glycol présentant de deux à huit atomes de carbone et d'un acide dicarboxylique aromatique.

3. Un dispositif selon la revendication 1 ou 2, dans lequel le téréphtalate polyalkylène glycol est choisi dans le groupe comprenant le téréphtalate polyéthylène glycol, le téréphtalate polypropylène glycol, le téréphtalate polybutylène glycol, et leurs copolymères.

4. Un dispositif selon l'une quelconque des dispositions précédentes dans lequel le polyester est choisi dans le groupe du poly(éthylènetéréphtalate), du poly(propylènetéréphtalate), et du poly(butylènetéréphtalate).

5. Un dispositif selon l'une quelconque des revendications précédentes, dans lequel le copolymère est un copolymère de téréphtalate polyéthylène glycol/poly(butylènetéréphtalate) copolymère.

6. Un dispositif selon l'une quelconque des revendications précédentes, dans lequel le copolymère comprend de vingt à quatre vingt dix pour cent en poids, de préférence de quarante à soixante dix pour cent en poids, de téréphtalate polyéthylène glycol.

7. Un dispositif selon l'une quelconque des revendications précédentes, dans lequel le téréphtalate polyalkylène glycol présente un poids moléculaire moyen de 150 à 4000, de préférence de 200 à 1500.

8. Un dispositif selon l'une quelconque des revendications précédentes, comprenant en outre un matériau qui facilite la croissance osseuse.

9. Un dispositif de fixation selon l'une quelconque des revendications précédentes, comprenant au moins un corps gonflant incluant ledit polymère.

10. Un dispositif de fixation selon la revendication 8 comprenant en outre des moyens de liaison pour relier des objets à ces moyens.

11. Un dispositif selon l'une quelconque des revendications précédentes, étant une vis pour os, une attache de suture, un bouchon pour le ligament antérieur croisé ACL (pour anterior cruciate ligament), une broche conique ou non conique ou une agrafe.

12. Un dispositif selon l'une quelconque des revendications précédentes, comprenant une partie de corps allongé s'étendant le long d'un axe central, la partie de corps allongé étant disposée sur une surface extérieure du corps munie d'un filetage de vis, le pas du filetage de vis s'étendant dans la direction axiale.

13. Un dispositif selon la revendication 12, dans lequel le corps allongé porte une partie de tête qui est munie d'au moins une surface d'entraînement destinée à venir en contact avec un outil d'entraînement.

14. Une attache de suture selon l'une quelconque des revendications 9 à 13, comprenant un multifilament ou un monofilament biodégradable pour relier du tissu musculaire ou ligamenteux.
